# EUROPEAN PATENT SPECIFICATION

(11) **EP 4 117 701 B1**
(45) Date of publication and mention of the grant of the patent: **01.07.2026**
(21) Application number: 21716236.1
(22) Date of filing: 09.03.2021
(51) Int. Cl.: A61K 36/899, A61K 36/53, A61K 31/7032, A61P 13/08

(54) **COMPOSITIONS FOR THE PREVENTION AND/OR TREATMENT OF BENIGN PROSTATIC HYPERTROPHY (BPH)**
ZUSAMMENSETZUNGEN ZUR PRÄVENTION UND/ODER BEHANDLUNG VON BENIGNER PROSTATAHYPERTROPHIE (BPH)
COMPOSITIONS POUR LA PRÉVENTION ET/OU LE TRAITEMENT DE L'HYPERPLASIE BÉNIGNE DE LA PROSTATE (HBP)

(30) Priority: 10.03.2020 IT 202000005053
(43) Date of publication of application: 18.01.2023
(73) Proprietor: IDI Integratori Dietetici Italiani S.r.l., 95020 Aci Bonaccorsi (CT) (IT)
(72) Inventor: BOTTINO, Alessandro, 95020 Aci Bonaccorsi (CT) (IT)
(74) Representative: Di Giovine, Paolo
(86) International application number: PCT/IB2021/051938
(87) International publication number: WO 2021/181258

(56) References cited:
- WO-A1-2017/108907
- CAMPOS MARIA GRAÇA ET AL: "What is the future of Bee-Pollen?", JOURNAL OF APIPRODUCT AND APIMEDICAL SCIENCE, vol. 2, no. 4, 1 October 2010 (2010-10-01), pages 131 - 144, XP093305796, ISSN: 1759-7986, DOI: 10.3896/IBRA.4.02.4.01
- PATERNITI IRENE ET AL: "Effects of different natural extracts in an experimental model of benign prostatic hyperplasia (BPH)", INFLAMMATION RESEARCH, BIRKHAEUSER VERLAG, BASEL, CH, vol. 67, no. 7, 20 April 2018 (2018-04-20), pages 617 - 626, XP036526316, ISSN: 1023-3830, [retrieved on 20180420], DOI: 10.1007/S00011-018-1152-9
- HARRY G. PREUSS ET AL: "A Critical Review of Cernitin(TM) for Symptomatic Relief Of Lower Urinary Tract Symptoms (LUTS) in Men", RESEARCH COMMUNICATIONS IN PHARMACOLOGY AND TOXICOLOGY, 1 January 2013 (2013-01-01), pages FP, 7-15, XP055450115
- FAGELMAN E ET AL: "HERBAL MEDICATIONS IN THE TREATMENT OF BENIGN PROSTATIC HYPERPLASIA (BPH)", UROLOGIC CLINICS OF NORTH AMERICA, SAUNDERS CO., LONDON, GB, vol. 29, no. 1, 1 February 2002 (2002-02-01), pages 23 - 29, XP001117401, ISSN: 0094-0143, DOI: 10.1016/S0094-0143(02)00015-0
- DI PAOLA R ET AL: "Teupolioside, a phenylpropanoid glycosides of Ajuga reptans, biotechnologically produced by IRBN22 plant cell line, exerts beneficial effects on a rodent model of colitis", BIOCHEMICAL PHARMACOLOGY, ELSEVIER, US, vol. 77, no. 5, 1 March 2009 (2009-03-01), pages 845 - 857, XP025941476, ISSN: 0006-2952, [retrieved on 20081125], DOI: 10.1016/J.BCP.2008.11.010

## Description

### FIELD OF THE INVENTION

The present invention relates to compositions comprising un pollen extract from Secale flowers *(Secale cereale L.)* and Teupolioside, preferably an extract of *Ajuga Reptans* titrated in Teupolioside. The present invention further relates to the use of such compositions for the prevention and/or treatment of Benign Prostatic Hypertrophy (BPH).

### STATE OF ART

Benign Prostatic Hypertrophy (BPH) is a pathology frequently associated to Lower Urinary Tract Symptoms (*LUTS*) such as dysuria, nycturia, urgency, difficulty in full bladder emptying, difficulty in starting urination, intermittent urination. BPH is characterized by the progressive increase in the prostate gland volume and by the worsening of the uroflowmeter parameters of the patients suffering therefrom. In BPH the prostate gland is subjected to hypertrophic-hyoperplastic phenomena of its own cell, epithelial and stromal components; the irritating and obstructive clinical symptoms deriving therefrom considerably interfere on the life quality of patients. Moreover, in the human tissue of BPH there are infiltrations of inflammatory cells and in the prostate tissue of subjects suffering from BPH an extended infiltration by activated T cells was noted (Zisman et al, Eur Urol 1999; Theyer et al, Lab Invest 1992). More than 50% of men over the age of 50 and more than 80% of men over the age of 80 report LUTS from BPH. First-line treatment in BPH are drugs, in particular alpha-lytic drugs (e.g., terazosin) and the inhibitors of 5-alphareductase (e.g., finasteride, dutasteride). In addition to drugs, further option is the surgical treatment; among the surgical treatments TURP (*Transurethral resection of the prostate*) represents the gold standard among the surgical treatments; among the other techniques: HoLEP (*Holmium laser enucleation of the prostate*), PUL (*Prostatic uretral lift*) can be mentioned. The use of food supplements is nowadays well-established in the treatment of BPH. *Serenoa repens* in particular is a small palm of arecaceae family - also known under the names of dwarf palm tree, sabal serrulata, or saw palmetto - endemic along the south-east coasts of United States. In *Serenoa repens* fruits several substances provided with pharmacological action have been identified, thereamong the phytosterols (plant molecules similar to cholesterol), such as beta-sitosterol. This molecule would be capable of inhibiting 5-alpha-reductase enzyme, enzyme capable of transforming testosterone in its active form, dihydrotestosterone (DHT). Considering that the prostate growth is physiologically stimulated by DHT, by inhibiting 5-alpha-reductase enzyme the prostate growth is reduced. The antiandrogen action of beta-sitosterol would be strengthened by the possibility of this molecule to act by interfering directly with the cellular receptor of DHT. Up to now there are several formulations indicated to improve BPH containing Serenoa repens. However, the treatments with these products not always are resolutive and they can have undesirable effects for the user. WO 2017/108907 A1 and PATERNITI IRENE ET AL: "Effects of different natural extracts in an experimentalmodel of benign prostatic hyperplasia (BPH)", INFLAMMATION RESEARCH, BIRKHAEUSER VERLAG, BASEL, CH, vol. 67, no. 7, 20 April 2018 (2018-04-20), pages 617-626, ISSN: 1023-3830, DOI: 10.1007/ S00011-018-1152-9 disclose *Ajuga reptans* extract (comprising teupolioside) for use in the treatment of benign prostatic hyperplasia (BPH), not disclosing a combination with a pollen extract from Secale cereale flowers.

HARRY G. PREUSS ET AL: "A Critical Review of Cernitin(TM) for Symptomatic Relief Of Lower Urinary Tract Symptoms (LUTS) in Men, RESEARCH COMMUNICATIONS IN PHARMACOLOGY AND TOXICOLOGY, 1 January 2013 (2013-01-01), page FP, 7-15," and FAGELMAN E ET AL: "HERBAL MEDICATIONS IN THE TREATMENT OF BENIGN PROSTATIC HYPERPLASIA (BPH)", UROLOGIC CLINICS OF NORTH AMERICA, SAUNDERS CO., LONDON, GB, vol. 29, no. 1, 1 February 2002 (2002-02-01), pages 23-29, ISSN: 0094-0143, DOI: 10.1016/ S0094-0143(02)00015-0 disclose, respectively, rye (i.e. Secale) flower pollen extract for treating LUTS (Lower Urinary Tract Symptoms) and BPH, but they do not disclose any combination with teupolioside.

DI PAOLA R ET AL: "Teupolioside, a phenylpropanoid glycosides of Ajuga reptans, biotechnologically produced by IRBN22 plant cell line, exerts beneficial effects on a rodent model of colitis", BIOCHEMICAL PHARMACOLOGY, ELSEVIER, US, vol. 77, no. 5, 1 March 2009 (2009-03-01), pages 845-857, ISSN: 0006-2952, DOI: 10.1016/J.BCP.2008.11.010 discloses the production of teupolioside by a cell line of *Ajuga reptans.*

Therefore, there is still the need for solutions for the prevention and/or treatment of benign prostatic hypertrophy.

### SUMMARY OF THE INVENTION

After extended experiments the inventors have found that the association of a pollen extract from Secale flowers *(Secale cereale L.*) and Teupolioside provides a natural and effective solution to the clinic problem of benign prostatic hypertrophy.

Therefore, the present invention relates to an association of such pollen extract and Teupolioside, preferably an extract of *Ajuga Reptans* titrated in Teupolioside will be used.

The present invention further relates to compositions, preferably for oral use, comprising the above-mentioned association and one or more suitable excipients and moreover to kit of portions including the above-mentioned active principles separated and formulated in suitable form of oral dosage for sequential or contemporary administration of the different active principles. The present invention further relates to the use of said association compositions and kit in the prevention and/or treatment of benign prostatic hypertrophy.

Thanks to its active ingredients, the composition set forth by the present invention allows to provide the patient an improvement in the disorders associated to BPH thanks to the effectiveness of the 2 active principles. Some advantages of the proposed invention are: single formulation which associates 2 active principles acting on BPH; ease of administration (e.g.: food supplement); not invasive, not painful treatment, high compliance of patient versus treatment, overcoming in terms of treatment effectiveness considering the nowadays available nutraceutical gold standard (e.g.: *Serenoa repens*)*.* Moreover, a phytotherapeutic treatment is desirable rather than the pharmacological treatments which in most cases are characterized by side effects and by poor compliance for patients.

Other advantages and features of the present invention will result evident from the following detailed description.

### BRIEF DESCRIPTION OF FIGURES

Figure 1. Cellular mechanism for testosterone-mediated production of the specific prostatic antigen 1.
   T: Testosterone; DHT Dihydrotestosterone; HSP: Heat-shock protein; AR: Androgen receptor; ARE: Androgen-responsive element.
Figure 2. Scheme of competition between the reaction catalysed by 5-α-reductase and the reduction in nitro blue tetrazolium (NBT) for the cofactor NADPH.
Figure 3. Effect of Dutasteride on the cell viability of the prostate cells LNCaP.
Figure 4. Effect of extracts of B. serrata (A), S. repens (B) and A. reptans (C) on the viability of the prostate cells LNCaP. * p<0.05.
Figure 5. Effect of treatment with the different plant extracts on the release of prostate-specific antigen (PSA) in prostate cells (LNCaP) stimulated with dihydrotestosterone (DHT). *p<0.05.
Figure 6. Effect of treatment with the extract of A. repens and S. repens on the activity of 5-α-reductase in prostate cells (LNCaP). * p<0.05.
Figure 7. Effect of solvent (A) and Diclofenac (B) on the cell viability of the prostate cells LNCaP. * p<0.05
Figure 8. Effect of extracts of B. serrata (A), S. repens (B) and A. reptans (C) on the viability of the prostate cells LNCaP. * p<0.05
Figure 9. IL-1β release profile after treatment of the prostate cells LNCaP with the plant extracts. * p<0.05
Figure 10. TNF-α release profile after treatment of the prostate cells LNCaP with the plant extracts. * p<0.05
Figure 11. Activity of total COXs (COX-1 and COX-2) in tumour prostate cells treated with the different plant extracts and Diclofenac. TC: Conditioned Medium. * p<0.05
Figure 12. Specific activity of COX-2 in tumour prostate cells treated with the different plant extracts and Diclofenac. TC: Conditioned Medium. * p<0.05

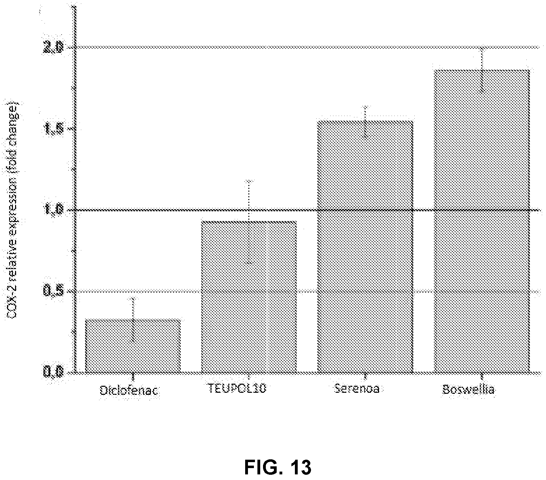

Figure 13. Relative expression of COX-2 in inflamed tumour prostate cells after treatment with Diclofenac and extracts of A. reptans (TEUPOL10), S. repens (Serenoa) and B. serrata (Boswellia) with respect to conditioned control. The black line identifies an expression level equal to the conditioned control.

Above the red line a significantly higher expression than the conditioned control is noted, whereas below the green line a significantly lower expression level than the conditioned control is noted.

### GLOSSARY

The terms used in the present description are as generally comprised by the person skilled in the art, except where differently designated.

Under the term "*extract*", in the context of the present description, any product is meant which can be traceable to a plant drug including all products derived from mechanical treatments (pulverization, trituration, mixing and/or other methods) or from extractive treatments (extraction with solvent, distillation and/or other specific methods) performed on a drug.

BPH: acronym of Benign Prostatic Hypertrophy or Hyperplasia or a condition characterized by the volume increase in the prostate gland; the term hypertrophy designates the volume increase of the single cells composing an organ, which keep unchanged their number, whereas hyperplasia designates the increase in number of cells.

5-alpha-reductase: an enzyme belonging to the class of oxidoreductase, which is capable of converting testosterone, the male sexual hormone, into dihydrotestosterone; in man, the enzyme is present in two isoforms, known as 5-alpha-reductase 1 and 2;
DHT: acronym of Dihydrotestosterone, is a biologically active metabolite of testosterone hormone, formed in the prostate, in the testicles, in the hair follicles and in the adrenal glands by 5α-reductase enzyme. Dihydrotestosterone belongs to the class of compounds called androgens; the androgens stimulate and control the development and maintenance of male features. DHT is considered to be approximatively three times as powerful as testosterone due to the higher affinity for the androgen receptors. Cyclooxygenase: is an enzyme, belonging to the class of oxidoreductase, which catalyses the conversion of arachidonic acid (a polyethenoid fatty acid with 20 atoms of carbon) in prostaglandin endoperoxide. Two distinct but correlated isoenzymes of COX exist: COX-1 is expressed constitutively on many tissues, and COX-2, instead, is inducible: its expression in the immune cells and of inflammation increases from 10 to 18 times after stimulation by growth factors, tumour promoters, cytokines, bacterial substances and thrombin. Cyclooxygenases are responsible for the synthesis of prostaglandins, endogenous autacoids (hormones with localized action) assigned to the adjustment of several biological phenomena, thereamong inflammation, pain sensitivity, fever, secretions of digestive organs, filtration of liquids at the renal level, reproduction and blood coagulation stand out.

Lipoxygenase: enzyme belonging to the class of oxidoreductase capable of producing hydroperoxides conjugated through oxidation of polyunsaturated fatty acids. It catalyses the first reaction of the so-called pathway of lipoxygenase, involved in the response of the plant organism to trauma and external stresses.

### DETAILED DESCRIPTION OF THE INVENTION

As mentioned above, the present invention relates to an association which comprises as active principles Teupolioside and a pollen extract from secale plant flowers *(Secale cereale L.)* Hereinafter the main active ingredients of the invention association are described.

Teupolioside is a molecule with the following structure:

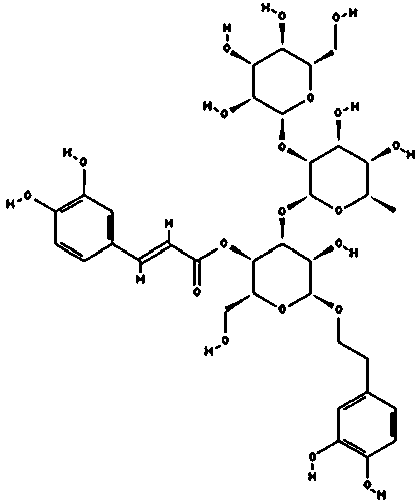

Teupolioside can be obtained from extracts of Ajuga reptans. Ajuga reptans (also known as bugle, St. Lawrence herb or Lorenza herb) belongs to the family of Lamiaceae consisting of about 300 native species of Europa, Asia and Africa, imported subsequently in Oceania and North America. The most important active ingredient obtained from Ajuga reptans indeed is phyenylpropanoid Teupolioside. Teupolioside (also known as Lamiuside "A") is a secondary metabolite produced by the plant for defensive purposes above all to protect from UV radiations. It was demonstrated that teupolioside has a wide variety of biological activities; its main activities are anti-inflammatory activity and activity on 5 alpha-reductase which is capable of modulating by acting consequently on the reduction of BPH evolution.

The extract of Ajuga reptans can be prepared for internal use through the preparation of infusions, decoctions or food supplements based on its leaves, dehydrated or the aerial portion of the plant (above all flowers), so as to exploit at best its most important curative qualities, such as the anti-inflammatory and astringent ones, very useful in case of intestinal and digestive disorders, thanks to its active principles, such as tannins, saponins, flavonoids, colin and bitter substances.

The extraction of Teupolioside from the whole plant through classical methods is not easy, since it is produced in quite reduced amounts. According to an embodiment Teupolioside will be obtained from suspension cell cultures of Ajuga reptans, in particular this product extracted from cell cultures is commercially available from the firm ABResearch.

The herein expressed percentages are meant as percentages by weight w/w. According to an embodiment an extract of Ajuga reptans with a titration from 10 to 50 % in teupolioside, preferably mixed with maltodextrins, will be used. For example, preferably an extract of Ajuga reptans with a titration in teupolioside at 10% or at 25% will be used, as those commercially available under the name Teupol 10P and Teupol 25P, respectively.

Apart from Teupolioside, the other active ingredient of the present invention is a pollen extract from Secale flowers (*Secale cereale L.,* Rye Grass)

According to an embodiment the extract includes a mixture of the water-soluble and liposoluble fractions of the pollen grain. The mixture can be prepared by mixing a raw material in powder which includes the isolated fraction soluble in water of the pollen grain with the raw material in paste which contains the liposoluble isolated portion of the pollen grain.

According to an embodiment an active ingredient of new implementation containing the water-soluble isolated fraction of the pollen grain with a concentration in amino acids of at least 6% (Graminex G60^{®} Flower Pollen Extract^{™}) and/or the liposoluble isolated fraction of the pollen grain with a concentration in phytosterols of at least 7% (Graminex NAX^{™} Flower Pollen Extract^{™}, raw material in paste) will be used.

The ratio between the water-soluble and liposoluble fraction preferably is at least 15:1.

According to an embodiment the extract will be prepared by mixing the product G60^{®} Flower Pollen Extract^{™} with the product NAX 7% Paste Flower Pollen Extract^{™}, preferably in ratio 15:1 (such ratio favours NAX component containing phytosterols). The increase in the phytosterol portion increases anti-inflammatory effectiveness of the extract in BPH. Both mentioned products are commercially available from Graminex^{®}. This new, ad hoc created mixture results to be particularly advantageous.

The present invention further relates to compositions comprising the association according to any one of the herein described embodiments and one or more suitable excipients.

According to an embodiment the composition comprises as active principles Teupolioside and an extract consisting of a mixture of pollen from Secale flowers *(Secale cereale L.)* as single active principles for use in the prevention and/or treatment of Benign prostatic hypertrophy.

Therefore, the composition can include other components, such as for example excipients, carriers, stabilizers, preservatives and the like.

The compositions according to any one of the embodiments provided in the present description can be formulated in any form and by any administration route and associated to any other component, in a variety of ways.

According to an embodiment, the compositions of the invention are compositions for oral use in solid form such as, for example, powder, orosoluble powder, granulate, hard capsule or soft-gel, tablet, sachet, pill or gelatin or in liquid form such as, for example, emulsions, solutions, prepared or extemporary suspensions, syrups and elixir, hard capsules, soft capsules, granulate, powder stick pack, liquid stick pack, powder for oral suspension, elixir, syrup, emulsion, tablets, pearls, chewing gum, chewable tablets, effervescent tablets.

Suitable excipients can be selected from those usually known in the state of art and include, but they are not limited thereto: diluents (for example dibasic calcium phosphate, lactose, microcrystalline cellulose and cellulose derivatives), thickeners (for example gums, hydroxypropylmethylcellulose and other cellulose derivatives), sweeteners (for example sorbitol, mannitol and other polyols, acesulfame K, aspartame, cyclamates, saccharin, sucralose), lubricants (for example magnesium stearate, stearic acid, waxes), dispersants, surfactants (for example sodium lauryl sulfate and polysorbates), flavouring agents, adsorbents (for example silica gel, talcum, starch, bentonite, kaolin), glidants and anti-adherent agents (for example talcum, colloidal silica, maize starch, silicon dioxide), colouring agents (for example titanium dioxide, iron oxides), opacifiers (for example titanium oxide), antioxidants, binders (for example gums, starch gelatin, cellulose derivatives, sucrose, sodium alginate), disaggregating agents (starch, microcrystalline cellulose, alginic acid, crospovidone), plasticizers (for example ethylcellulose and other cellulose derivatives, acrylates and methacrylates, glycerol and sorbitol), preservatives (for example parabens, sulphur dioxide), viscosifiers, emulsifiers, humectants, bathers, chelating agents and mixtures thereof.

The above-mentioned solid dosage forms could be coated with enteric, gastric coatings or other type known in the state of art. They can allow the release of the active ingredients only or preferably in a certain tract of intestine, in case, in delayed time. Substances which can allow such delayed use include, but they are not limited thereto, polymers and waxes.

The compositions of the present invention will be, for example, a medical device, a food supplement, a nutraceutical, dietetic or nutritional composition, food product, a beverage, food, a nutraceutical, medicated food, food for special medical purposes, or a pharmaceutical composition, a homeopathic product, a herbal medicine, a drug.

The compositions could be administered for example with a dosage regime comprised between 10.00 to 300,00 mg/day of Teupolioside and from 200,00 mg/day to 1000,00 mg/day of pollen extract.

The compositions could include for example between 10 and 300 mg of Teupolioside and/or between 40 and 1200 mg of ajuga reptans extract titrated between 10 and 50 % in Teupolioside and/or between 200 and 1000 mg of a pollen extract from Secale flowers *(Secale cereale L.).* The combination of the above-mentioned active principles could be used formulated in one single composition according to the several above-described embodiments or in a kit which comprises the several separated ingredients, for example, in single compositions formulated in suitable oral dosage form as above defined for the sequential or contemporary administration of the different active principles. Therefore, the present invention further relates to kit of portions which include the different active principles of the association according to any one of the herein described embodiments separated and formulated in suitable oral dosage form for sequential or contemporary administration of the different active principles.

Moreover, the present invention further relates to the association, to the compositions and to the kit, according to any one of the herein described embodiments, for use in the prevention and/or treatment of symptoms and/or disorders associated to BPH. The association of the above-mentioned active principles could be formulated in one single composition or in a kit according to the various above-described embodiments and prepared for example by mixing the selected active principles with possible other excipients as known to the person skilled in the art.

### EXAMPLES

Hereinafter some not limitative examples of the compositions according to the present invention are reported. In the examples the percentages, if present, are to be meant as percentages by weight. Modifications or variations of the herein exemplified embodiments, obvious to a person skilled in the art, are comprised by the enclosed claims.

Graminex^{®} and G96^{™} Flower Pollen Extract^{™} are registered trademarks Graminex LLC used under licence.

### EXAMPLE 1:

| *Ingredient* | *Amount per single dosage unit (%)* 15 |
|---|---|
| Graminex^{®} G96 Flower Pollen Extract^{™} - Pollen extract from Secale flowers (*Secale cereale L.*) | 58.140 |
| Microcrystalline cellulose | 30.042 |
| Teupol 25 P - Extract of Ajuga reptans from cell cultures | 4.651 |
| Mono and diglycerides of fatty acids | 1.977 |
| Hydroxypropylmethylcellulose (Coating agent) | 1.628 |
| Titanium dioxide (colouring agent) | 0.977 |
| Magnesium salts of fatty acids | 0.967 |
| Silicon dioxide | 0.967 |
| Talcum (Coating agent) | 0.488 |
| Polyethylene glycol (Coating agent) | 0.163 |

Pharmaceutic form: tablet

### EXAMPLE 2:

| *Ingredient* | *Amount per single dosage unit (%)* |
|---|---|
| Graminex^{®} G96 Flower Pollen Extract^{™} - Pollen extract from Secale flowers (*Secale cereale L*.) | 58.140 |
| Microcrystalline cellulose | 27.716 |
| Teupol 25 P - Extract of Ajuga reptans from cell cultures | 6.977 |
| Mono and diglycerides of fatty acids | 1.977 |
| Hydroxypropylmethylcellulose (Coating agent) | 1.628 |
| Titanium dioxide (colouring agent) | 0.977 |
| Magnesium salts of fatty acids | 0.967 |
| Silicon dioxide | 0.967 |
| Talcum (Coating agent) | 0.488 |
| Polyethylene glycol (Coating agent) | 0.163 |

### Pharmaceutical form: tablet

### Experimental evidence

### IN-VITRO STUDIES

The effectiveness of the extract of Ajuga reptans was verified on human fibroblasts (LNCaP) and compared with the action of Serenoa Repens, plant traditionally used for the treatment of BPH. The results showed that the extract of ajuga reptans is capable of inducing an inhibition of 85% on 5 alpha-reductase enzyme, with respect to 48% of the cells treated with serenoa repens.

### COMPARATIVE EVALUATION OF THE EFFECTIVENESS OF TEUPOLIOSIDE 10P IN THE REDUCTION OF PSA AND ACTIVITY OF 5-Alfa-REDUCTASE

### Aim

The aim of the present study is to evaluate the effect on the release of prostate-specific antigen (PSA) and on the activity of 5-α-reductase at the prostate level of a new extract of Ajuga reptans (TEUPOL10), by comparing it with plant extracts, respectively of Boswellia serrata and Serenoa repens, already used in commercially available formulations.

### Experimental procedures

### Determination of cytotoxicity of plant extracts

The cytotoxic activity of considered plant extracts was evaluated in a human prostate cell line (LNCaP). The prostate cells were treated with growing concentrations of the plant extracts (from 0 to 1000 µg/mL for the extract of B. Serrata and S. Repens, from 0 to 1500 µg/mL for TEUPOL10) and of the positive control Dutasteride (from 0 µg/mL to 7.93 µg/mL). The treatment was prolonged for 24 hours, time corresponding to the overall duration of the experiments related to PSA and to 5-α-reductase. At the end of the incubation period, the vitality of the prostate cells was evaluated by using the assay MTS, based upon the reduction of the compound MTS tetrazolium by the vital cells to generate a coloured form, which can be quantified by measuring the absorbance thereof at 490-500 nm.

### Determination of the release of prostate-specific antigen (PSA)

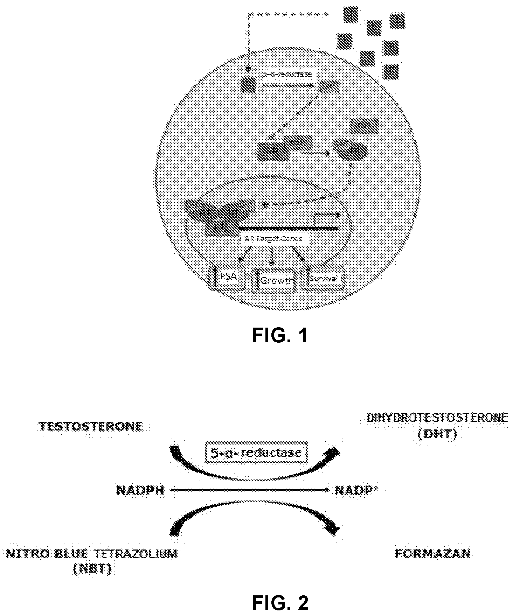

The release of PSA after treatment with the plant extracts was evaluated in prostate cells (LNCaP). With the purpose of treating the cells with the same amount of active principle, the concentrations of treatment of the extracts were corrected, by considering the different percentage of active principle existing in the same (65% boswellic acids in the extract of B. serrata, 30% of total fatty acids in the extract of S. *repens* and 10% of teupolioside in the extract of A. reptans, respectively) (Table 1). After having sowed the prostate cells and waited for 48 hours to obtain the maximum adhesion thereof, the culture medium was replaced by medium without hormones. The next day, LNCaPs were treated with Dutasteride and the different extracts, in presence or absence of dihydrotestosterone (DHT, 10 nM), an androgen hormone which stimulates the release of PSA. As shown in Figure 1, such androgen was added to eliminate possible interferences linked to the activity of 5-α-reductase, enzyme responsible for the conversion of testosterone into dihydrotestosterone.

At the end of the incubation period (24 hours), the supernatants were recovered and the release of PSA after treatments quantified by assay ELISA (Enzyme-Linked Immunosorbent Assay). The obtained results were normalized on the viability of the prostate cells, evaluated by using the previously described assay MTS.

**Table 1. Concentrations of treatment of plant extracts and of Dutasteride.**

| **Compound** | **Extract concentration (µg/mL)** | **Active ingredient concentration (µg/mL)** |
|---|---|---|
| Dustasterid | 5.3 | 5.3 |
| Extract of *B.serrata* | 25 | 16.3 |
| Extract of *S.repens* | 54.2 | 16.3 |
| Extract of *A.reptans* (TEUPOL10) | 162.5 | 16.3 |

### Determination of activity of 5-α-reductase

5-α-reductase enzyme, existing at the level of prostate epithelium, catalyses the reduction of testosterone into dihydrotestosterone (DHT), hormone provided with a higher activity than testosterone itself. The activity of 5-α-reductase was determined in prostate cells by using a colorimetric method, based upon the competition for the cofactor NADPH between 5-α-reductase and the colouring substrate (Figure 2). After sowing, adhesion and replacement of the culture medium with a medium without hormones, the prostate cells were treated for 24 hours in presence of testosterone (5 µM) and of the extracts at the concentrations shown in Table 1. At the end of incubation, the cells were washed and exposed for 5 hours to the development buffer containing, apart from testosterone and other extracts, Nitro Blue Tetrazolium (NBT). The salt of coloured tetrazolium, accumulated inside the cells and proportional to the inhibition of the activity of 5-α-reductase, was quantified, after lysis of cells, by measuring the absorbance thereof at 570 nm. At last, the obtained absorbances were normalized for the protein content of the cell lysates, determined spectrophotometrically at 280 nm.

### Results

Cytotoxic effect of the plant extracts The cytotoxic activity of the positive control (Dutasteride) and of the plant extracts of B. serrata (Boswellia), S. repens (Serenoa) and A. reptans (TEUPOL10) was evaluated on prostate cells (LNCaP).

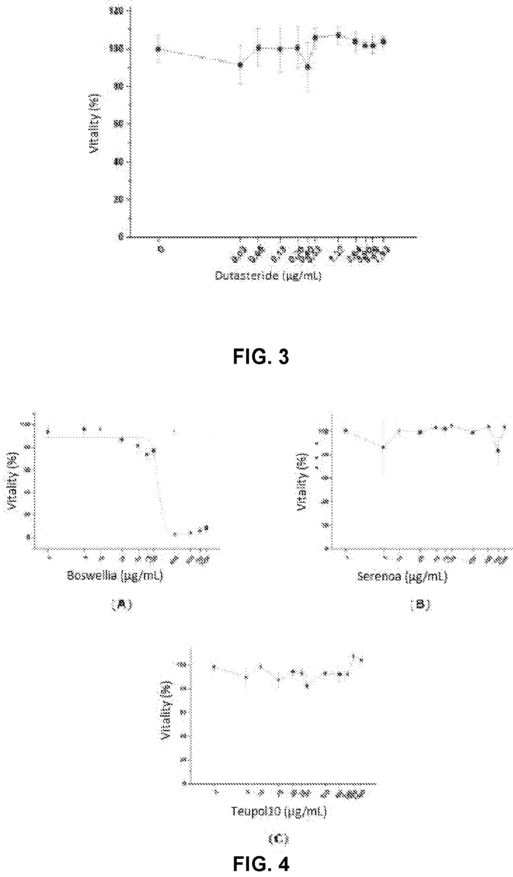

No adverse effect was observed for Dutasteride at all tested concentrations (Figure 2). For the solvent (DMSO) concentrations lower than the toxicity limit shown in literature for periods of time higher than 24 hours were used. As it can be seen from Figure 3, the extract of B. serrata only (Figure 4A) causes a slight reduction in the vitality of the prostate cells starting from the concentration of 50 µg/mL. No adverse effect was observed after treatment with extract of S. repens and of A. reptans (Figure 4B and 4C) at all wished concentrations.

### Effect of treatment with plant extracts on the release of prostate-specific antigen (PSA)

Prostate-specific antigen (PSA) is a protein released by the prostate epithelium and the increase in its blood concentration is associated to the development of prostate pathologies such as Benign Prostatic Hypertrophy (BPH). PSA, then, represents an optimum parameter for analysing the impact of a substance at prostate level.

As it can be seen from Figure 4, TEUPOL10 and B. serrata inhibit significantly the production of PSA in prostate cells (LNCaP) stimulated with DHT, respectively by 15% and by 12%. No reduction was observed for S. repens. The reduction in the release of PSA by the extract of B. Serrata and Dutasteride is linked to alterations in the expression of androgen receptor and of PSA, both passages downstream of the DHT production.

As expected, the stimulation with DHT brought to an increase in the release of PSA by the prostate cells (Figure 4).

### Effect of treatment with plant extracts on the activity of 5-α-reductase

As seen previously (Figure 1), the production of prostate-specific antigen (PSA) is linked to the presence of dihydrotestosterone (DHT). The latter is produced starting from testosterone by means of a reduction reaction catalysed by 5-α-reductase, a key enzyme in the occurrence and development of many prostate pathologies. Consequently, as for PSA release, the inhibition of the activity of 5-α-reductase, by a substance such as a plant extract, can indicate a beneficial effect at prostate level, both in preventive terms and in terms of mitigating the symptoms.

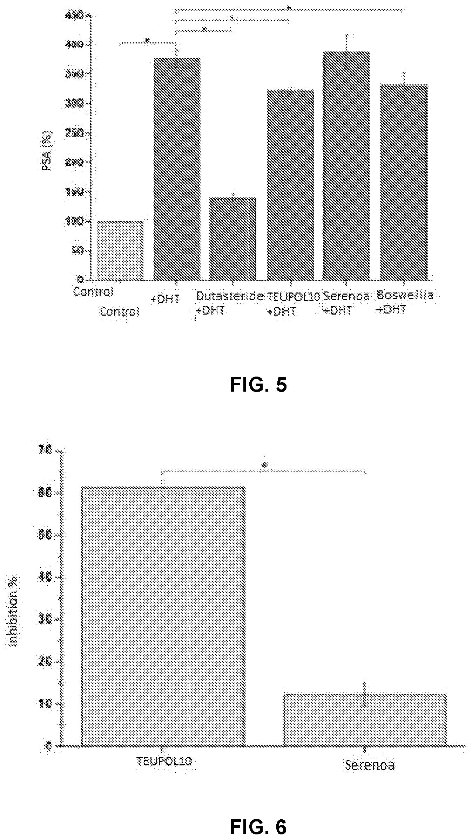

As it can be seen in Figure 6, TEUPOL10 has an inhibitory effect on the activity of 5-α-reductase significantly higher than the extract of S. repens (61% and 12% respectively).

### CONCLUSIONS

The extract of A. reptans (TEUPOL10) reduces the release of prostate-specific antigen (PSA) significantly higher than the extract of S. repens. Moreover, TEUPOL10 results to be more effective than the extract of S. repens in inhibiting the activity of 5-α-reductase, key enzyme in the occurrence and development of Benign Prostatic Hypertrophy (BPH).

### EVALUATION OF THE ANTI-INFLAMMATORY EFFECTIVENESS

### Aim

The aim of the present study is to evaluate the specific anti-inflammatory activity at the prostate level of a new extract of Ajuga reptans (TEUPOL10), by comparing it with plant extracts, of Boswellia serrata and Serenoa repens, respectively, already used in commercially available formulations.

### Experimental procedures

### Determination of cytotoxicity of plant extracts

The cytotoxic activity of the considered plant extracts was evaluated in a human tumour prostate cell line (LNCaP). The prostate cells were treated with growing concentrations of the plant extracts (da 0 a 1000 µg/mL for the extract of B. serrata and S. repens, from 0 to 1500 µg/mL for TEUPOL10), of solvent DMSO (from 0 to 5% (V/V)) and of the positive control of anti-inflammatory activity, Diclofenac (from 0 to 1590 µg/mL). The treatment was prolonged for 6 hours, period of time corresponding to the overall duration of the pre-treatment and of post-treatment thereto the cells were subjected for the subsequent evaluation of the anti-inflammatory activity. At the end of the incubation period, the vitality of the prostate cells was evaluated by using the assay MTS, based upon the reduction of the compound MTS tetrazolium by the vital cells to generate a form of coloured formazan, which can be quantified by measuring the absorbance at 490-500 nm thereof.

### Specific prostate anti-inflammatory activity

The specific anti-inflammatory activity at prostate level of the plant extracts was evaluated in tumour prostate cells (LNCaP). In order to treat the cells with the same amount of active principle, the concentrations of treatment of extracts were corrected, by considering the different percentage of active principle existing in the same (65% boswellic acids in the extract of B. serrata, 30% of total fatty acids in the extract of S. repens and 10% of teupolioside in the extract of A. reptans, respectively). The treatment protocol consists of two steps: 1) pre-treatment step, lasting 2 hours, in which the cells were treated with the extracts and Diclofenac at the concentration shown in Table 1 and 2) post-treatment step (4 hours) in which the cells were stimulated with conditioned culture medium THP-1 (TC, inflammatory stimulus) after treatment with LPS (lipopolysaccharide), in presence or absence of plant extracts. The overall treatment time (6 hours) makes reference to plasma elimination half-life of 11-keto β-boswellic acid as shown in work of Sharma et al., 2004

**Table 2. Concentrations of treatment of plant extracts and Diclofenac.**

| **Compound** | **Raw material concentration (µg/mL)** | **Active ingredient concentration (µg/mL)** |
|---|---|---|
| **Diclofenac** | 0.03 | 0.03 |
| **Extract of *B.serrata*** | 0.1 | 0.06 |
| **Extract of *S.repens*** | 0.2 | 0.06 |
| **Extract of *A.reptans* (TEUPOL10)** | 0.6 | 0.06 |

Cells stimulated exclusively with the conditioned culture medium THP-1 for the duration of the treatment step were used as inflammation positive control whereas cells treated with Diclofenac in presence or absence of the conditioned culture medium THP-1 were used as positive control of anti-inflammatory activity. At the end of each step images of the cells were acquired to evaluate possible morphological changes. The anti-inflammatory activity of the plant extracts was evaluated by quantifying the pro-inflammatory cytokines IL-1β and TNF-α released after treatments by assay ELISA (*Enzyme-Linked Immunosorbent Assay*)*.*

### Determination of the activity and of the expression of cyclooxygenases (COX-1 e COX-2)

The anti-inflammatory effect of the plant extracts was confirmed by measuring the activity of cyclooxygenase (COX) 1 and 2. The total activity of COXs, as well as the specific activity of COX-2, was evaluated by using a kit. With the purpose of evaluating the gene expression of COXs, the RNA present in the cells subjected to the different treatments was extracted and purified to limit the contamination from genomic DNA, by using a commercial kit. The good quality of RNA was confirmed by means of electrophoresis on 1% agarose gel, by observing the two characteristic bands attributable to RNA. After quantification, RNA was reverse transcribed by using a commercial kit. Before proceeding with analysing the expression of COXs by means of qPCR (quantitative PCR), the occurred reverse transcription by PCR was verified by using primers for standard β-actin and by verifying electrophoretically the presence of the amplification product by means of 2% agarose gel. For the analysis of expression of COXs by means of qPCR (Roche Lightcycler 480 II), the gene GADPH (Glyceraldehyde 3-phosphate dehydrogenase) was selected as reference (housekeeping), as shown in Silvestri et al., 2013. At the end of qPCR, the obtained data were analysed by calculating the relative expression of the genes of interest towards the reference housekeeping gene (fold change), through the formula 2exp-ΔΔCt, according to the method shown in Livak and Schmittgen, 20013. The expression of the genes of interest is considered significantly increased with respect to the housekeeping when the fold change is higher than 2, and significantly reduced with a fold change lower than 0.5. The presence of the amplicons (GADPH 122 bp, COX-1 60 bp and COX-2 75 bp) was verified by electrophoresis in 2% agarose gel.

### Results

### Cytotoxic effect of plant extracts.

The cytotoxic activity of the solvent (DMSO), of the positive control of anti-inflammatory activity (Diclofenac) and of the plant extracts of B. Serrata (Boswellia), S. Repens (Serenoa) and A. Reptans (TEUPOL10) was evaluated on tumour prostate cells (LNCaP).

The vitality of the prostate cells at the end of 6 hours of treatment decreases significantly at the concentration of 0.5% (V/V) for DMSO and of 80 µg/mL for Diclofenac (Figure 1). On the contrary, as it can be seen from Figure 2, the extract of B. serrata only (Figure 2A) causes a decrease in the vitality of the prostate cells starting from the concentration of 250 µg/mL whereas no adverse effect was observed after treatment with extract of S. repens and of A. reptans (Figure 2B and 2C) at all considered concentrations.

### Anti-inflammatory activity of plant extracts

### Effect of treatment with plant extracts on the release of pro-inflammatory cytokines (IL-1B and TNF-α)

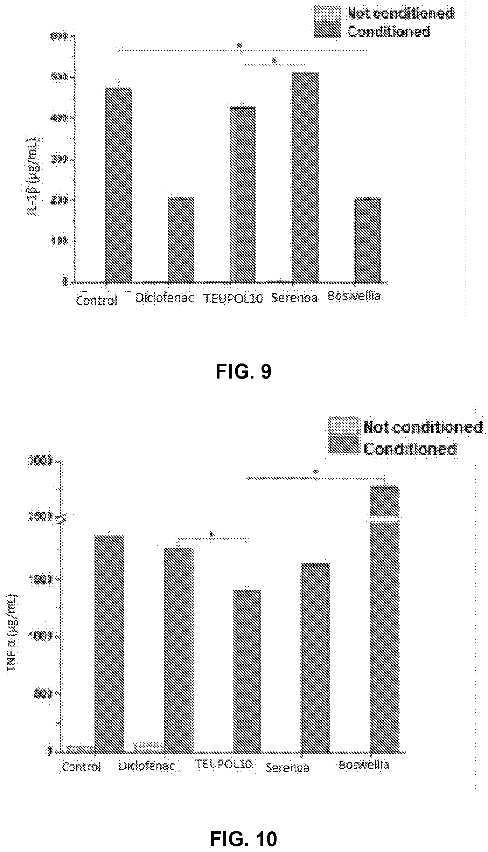

The specific prostatic anti-inflammatory activity was evaluated on tumour prostate cells (LNCaP) by measuring the level of pro-inflammatory cytokines IL-1β and TNF-α released in the culture medium in presence or absence of plant extracts. As it can be seen from Figure 9, the treatment with TEUPOL10 reduces significantly the released amount of IL-1β with respect to the cells treated with the conditioned medium (about 10%) and with the extract of S. repens (about 20%).

Contrary to what happens for IL-1β, in which the maximum reduction is observed after treatment with Diclofenac and the extract of B. serrata, the release of TNF-α is inhibited more effectively by TEUPOL10, showing better performance, not only with respect to other extracts, but even compared to Diclofenac (Figure 10). No morphological changes of the tumour prostate cells after treatment with the considered extracts were observed.

### Impact of the treatment with plant extracts on the total activity of cyclooxygenase (COX-1 and COX-2) and on the specific activity of COX-2

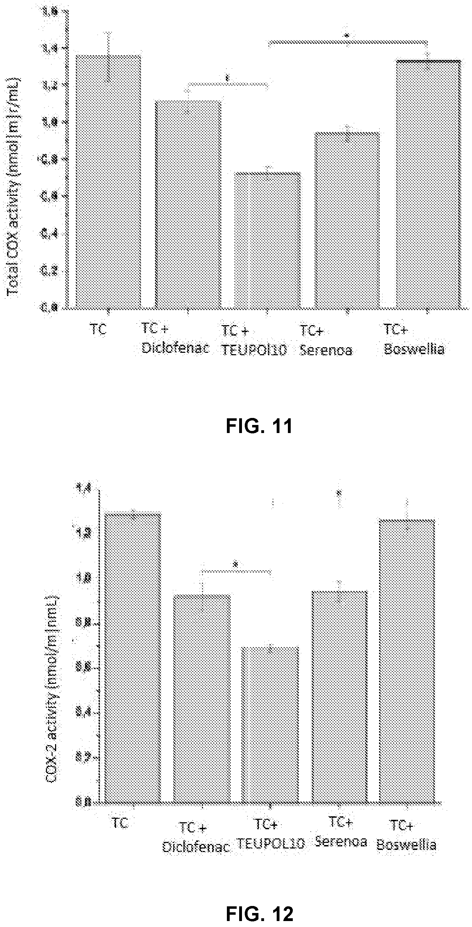

As shown in Figure 11, the activity of total COXs reduces significantly after treatment with Diclofenac and with the plant extracts. Among the latter, TEUPOL10 has the maximum reduction in the activity of total COXs (about 50%), higher than Diclofenac. The same inhibition profile was observed even for the specific activity of COX-2 (Figure 6), specific cyclooxygenase of cells and inflamed tissues, confirming the effective anti-inflammatory activity of TEUPOL10.

### Analysis of the expression of cyclooxygenase, COX-1 and COX-2, after treatment with the plant extracts

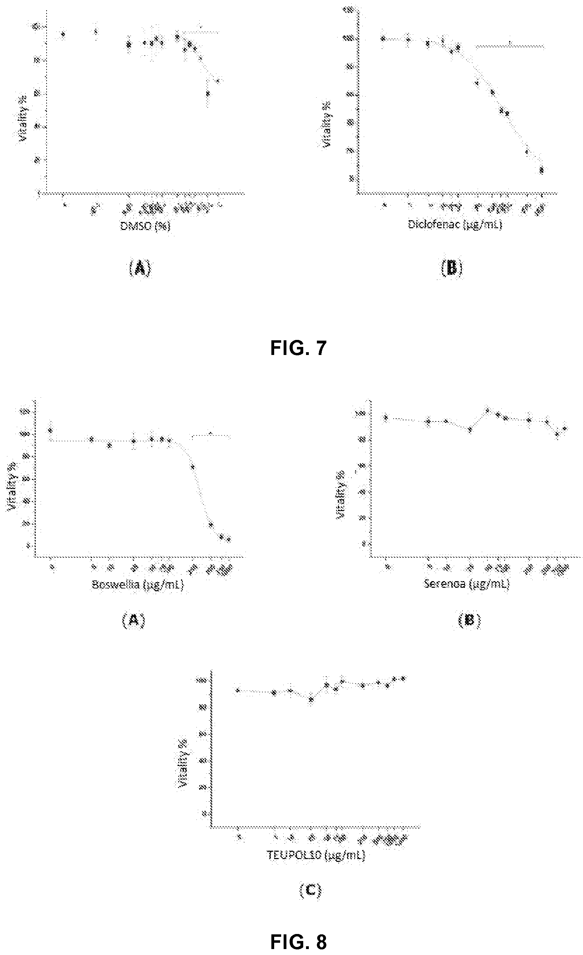

In order to determine the effect of treatment with the plant extracts on the expression of typical genes of the anti-inflammatory response, the expression of COX-1, isoform of constitutively expressed cyclooxygenase, and COX-2, the expression thereof increases in cells and inflamed tissues, were analysed. According to the work of Silvestri et al., 2013, no expression of COX-1 was observed under all considered conditions, showing a low level of expression of the constituting cyclooxygenase. As it can be seen in Figure 7, the expression of the inducible form of cyclooxygenase (COX-2), against the tumour prostate cells treated with il conditioned medium, remains unaltered after treatment with all plant extracts with respect to the inflamed control, whereas a significant decrease is observed after treatment with Diclofenac.

### Conclusions

The extract of A. reptans TEUPOL10, the active concentration (0.06 mg/mL) being equal, has a specific anti-inflammatory activity at prostate level higher than other considered plant extracts (B. serrata and S. repens). In detail, TEUPOL10 reduces significantly more the release of pro-inflammatory cytokines IL-1β and TNF-α with respect to the other extracts and Diclofenac. The effective anti-inflammatory activity is further confirmed by the significant reduction of the total COXs and of COX-2, even if the gene expression of the latter is not modified. At last, TEUPOL10, at the considered concentrations, has no adverse effects on the vitality of the prostate cells, contrary to the extract of B. serrata and Diclofenac.

Client of the mentioned studies: ABResearch Srl. The used products were produced under licence from "IRB Spa - Altavilla Vicentina". The two active ingredients used in combination show an unexpected strengthening with respect to the two active ingredients used singularly.

## Claims

1. An association of Teupolioside and a pollen extract from Secale flowers (*Secale cereale L., Rye Grass)* for use in the prevention and/or the treatment of benign prostatic hypertrophy.

2. A composition comprising teupolioside and a pollen extract from Secale flowers (*Secale cereale L.,* Rye Grass) and one or more excipients.

3. The composition according to claim 2 wherein:
- the pollen extract is an amount of between 200 mg and 1000 mg, preferably between 300 mg and 500 mg per dosage unit; and/or
- teupolioside is an amount of between 10 mg and 300 mg and/or between 40 and 1200 mg of ajuga reptans extract titrated between 10 and 50% in teupolioside preferably between 10 mg and 15 mg of teupolioside or between 40 and 60 mg of ajuga reptans extract titrated at 25% in teupolioside per dosage unit.

4. The composition according to claim 2 or 3 for oral use.

5. The composition according to claim 4 in a form selected from powder, orosoluble powder, granulate, hard capsule or soft-gel, tablet, sachet, pill, gelatin, emulsion, solution, suspension, syrup, elixir, hard capsule, soft capsule, granulate, powder stick pack, liquid stick pack, powder for oral suspension, elixir, syrup, emulsion, tablets, pearls, chewing gum, chewable tablets, effervescent tablets.

6. The composition according to any one of claims 2 to 5 in the form of a medical device, food supplement, nutraceutical, dietetic or nutritional composition, food product, beverage, food, medicated food, food for special medical purposes, pharmaceutical composition, drug, homeopathic product, herbal medicine.

7. The composition according to any one of claims 2 to 6 comprising an extract of *Ajuga reptans,* preferably said extract is obtained from a suspension cell culture *Ajuga reptans.*

8. The composition according to claim 7 wherein said extract of *Ajuga reptans* is titrated at 10 to 50% of teupolioside, preferably it is titrated at 10 to 25% of teupolioside.

9. The composition according to any one of claims 2 to 8, wherein said pollen extract comprises a mixture of the water-soluble and liposoluble fractions of the pollen grain, preferably said mixture has a water-soluble and liposoluble fraction ratio of at least 15:1.

10. The composition according to claim 9, wherein said water-soluble fraction has a concentration of amino acids of at least 6% and/or said liposoluble fraction has a concentration of phytosterols of at least 7%.

11. The composition according to any one of claims 2 to 10 for use in the prevention and/or treatment of benign prostatic hypertrophy, preferably according to a dosage regime comprised between 10.00 and 300.00 mg/day of Teupolioside and/or between 200.00 mg/day and 1000.00 mg/day of pollen extract.

## Patentansprüche

1. Assoziation von Teupoliosid und einem Pollenextrakt aus Secale-Blüten (*Secale cereale L., Weidelgras*) zur Verwendung bei der Vorbeugung und/oder der Behandlung von gutartiger Prostatahypertrophie.

2. Zusammensetzung, umfassend Teupoliosid und einen Pollenextrakt aus Secale-Blüten *(Secale cereale L.,* Roggen) und einen oder mehrere Hilfsstoffe.

3. Zusammensetzung nach Anspruch 2, wobei:
- der Pollenextrakt eine Menge zwischen 200 mg und 1000 mg, vorzugsweise zwischen 300 mg und 500 mg, pro Dosierungseinheit beträgt; und/oder
- Teupoliosid eine Menge von zwischen 10 mg und 300 mg und/oder zwischen 40 und 1200 mg Ajuga-reptans-Extrakt, titriert auf zwischen 10 und 50 % Teupoliosid, vorzugsweise zwischen 10 mg und 15 mg Teupoliosid oder zwischen 40 und 60 mg Ajuga-reptans-Extrakt, titriert auf 25 % Teupoliosid, pro Dosierungseinheit beträgt.

4. Zusammensetzung nach Anspruch 2 oder 3 zur oralen Anwendung.

5. Zusammensetzung nach Anspruch 4 in einer Form ausgewählt aus Pulver, oral löslichem Pulver, Granulat, Hartkapsel oder Weichgel, Tablette, Sachet, Pille, Gelatine, Emulsion, Lösung, Suspension, Sirup, Elixier, Hartkapsel, Weichkapsel, Granulat, Pulver-Stickpack, Flüssigkeits-Stickpack, Pulver für eine orale Suspension, Elixier, Sirup, Emulsion, Tabletten, Perlen, Kaugummi, Kautabletten, Brausetabletten.

6. Zusammensetzung nach einem der Ansprüche 2 bis 5 in Form eines Medizinprodukts, Nahrungsergänzungsmittels, Nutrazeutikums, einer diätetischen oder Ernährungszusammensetzung, eines Nahrungsmittelprodukts, Getränks, Nahrungsmittels, medikamentösen Nahrungsmittels, Lebensmittels für besondere medizinische Zwecke, einer pharmazeutischen Zusammensetzung, eines Arzneimittels, homöopathischen Produkts, pflanzlichen Arzneimittels.

7. Zusammensetzung nach einem der Ansprüche 2 bis 6, umfassend einen Extrakt *aus Ajuga reptans,* wobei der Extrakt vorzugsweise erhalten wird aus einer Suspensionszellkultur von *Ajuga reptans.*

8. Zusammensetzung nach Anspruch 7, wobei der Extrakt von *Ajuga reptans* auf 10 bis 50 % Teupoliosid titriert ist, wobei er vorzugsweise auf 10 bis 25 % Teupoliosid titriert ist.

9. Zusammensetzung nach einem der Ansprüche 2 bis 8, wobei der Pollenextrakt eine Mischung der wasserlöslichen und fettlöslichen Fraktionen des Pollenkorns umfasst, wobei vorzugsweise die Mischung ein Verhältnis von wasserlöslicher zu fettlöslicher Fraktion von mindestens 15:1 aufweist.

10. Zusammensetzung nach Anspruch 9, wobei die wasserlösliche Fraktion eine Konzentration an Aminosäuren von mindestens 6 % aufweist und/oder die fettlösliche Fraktion eine Konzentration an Phytosterolen von mindestens 7 % aufweist.

11. Zusammensetzung nach einem der Ansprüche 2 bis 10 zur Verwendung bei der Prävention und/oder Behandlung von benigner Prostatahypertrophie, vorzugsweise gemäß einem Dosierungsschema, das zwischen 10,00 und 300,00 mg/Tag Teupoliosid und/oder zwischen 200,00 mg/Tag und 1000,00 mg/Tag Pollenextrakt umfasst.

## Revendications

1. Association de teupolioside et d'un extrait de pollen de fleurs de seigle (*Secale cereale L., Raye Grass*) pour une utilisation dans la prévention et/ou le traitement de l'hypertrophie bénigne de la prostate.

2. Composition comprenant du teupolioside et un extrait de pollen de fleurs de seigle (*Secale cereale L.,* Raye Grass) et un ou plusieurs excipients.

3. Composition selon la revendication 2, dans laquelle :
- l'extrait de pollen est présent en une quantité comprise entre 200 mg et 1000 mg, de préférence entre 300 mg et 500 mg par unité posologique ; et/ou
- le teupolioside est présent en une quantité comprise entre 10 mg et 300 mg et/ou entre 40 et 1200 mg d'extrait *d'Ajuga reptans* titré entre 10 et 50 % dans du teupolioside, de préférence entre 10 mg et 15 mg de teupolioside ou entre 40 et 60 mg d'extrait *d'Ajuga reptans* titré à 25 % dans du teupolioside par unité posologique.

4. Composition selon la revendication 2 ou 3 pour une utilisation par voie orale.

5. Composition selon la revendication 4 sous une forme choisie parmi poudre, poudre orosoluble, granulé, gélule ou capsule molle, comprimé, sachet, pilule, gélatine, émulsion, solution, suspension, sirop, élixir, gélule, capsule molle, granulé, stick de poudre, stick de liquide, poudre pour suspension orale, élixir, sirop, émulsion, comprimés, perles, chewing-gum, comprimés à mâcher, comprimés effervescents.

6. Composition selon l'une quelconque des revendications 2 à 5 sous la forme d'un dispositif médical, complément alimentaire, composition nutraceutique, diététique ou nutritionnelle, produit alimentaire, boisson, aliment, aliment médicamenteux, aliment destiné à des fins médicales spéciales, composition pharmaceutique, médicament, produit homéopathique, médicament à base de plante.

7. Composition selon l'une quelconque des revendications 2 à 6, comprenant un extrait *d'Ajuga reptans,* de préférence ledit extrait est obtenu à partir d'une culture cellulaire en suspension *d'Ajuga reptans.*

8. Composition selon la revendication 7, dans laquelle ledit extrait d*'Ajuga reptans* est titré à 10 à 50 % de teupolioside, de préférence il est titré à 10 à 25 % de teupolioside.

9. Composition selon l'une quelconque des revendications 2 à 8, dans laquelle ledit extrait de pollen comprend un mélange des fractions hydrosolubles et liposolubles du grain de pollen, de préférence ledit mélange a un rapport entre la fraction hydrosoluble et la fraction liposoluble d'au moins 15:1.

10. Composition selon la revendication 9, dans laquelle ladite fraction hydrosoluble a une concentration en acides aminés d'au moins 6 % et/ou ladite fraction liposoluble a une concentration en phytostérols d'au moins 7 %.

11. Composition selon l'une quelconque des revendications 2 à 10 pour une utilisation dans la prévention et/ou le traitement de l'hypertrophie bénigne de la prostate, de préférence selon un schéma posologique compris entre 10,00 et 300,00 mg/jour de teupolioside et/ou entre 200,00 mg/jour et 1000,00 mg/jour d'extrait de pollen.
